# EUROPEAN PATENT SPECIFICATION

(11) **EP 0 858 432 B1**
(45) Date of publication and mention of the grant of the patent: **02.11.2000**
(21) Application number: 95928923.2
(22) Date of filing: 30.08.1995
(51) Int. Cl.: C02F 1/32, A61L 2/10, B01J 19/12

(54) **ULTRAVIOLET STERILIZING SYSTEM FOR WASTEWATER**
UV-ENTKEIMUNGSSYSTEM FÜR ABWASSER
SYSTEME POUR LA STERILISATION PAR RAYONS ULTRAVIOLETS D'EAUX USEES

(43) Date of publication of application: 19.08.1998
(73) Proprietor: UV Systems Technology Inc., Burnaby, British Columbia V5C 6G7 (CA)
(72) Inventor: FREE, David, Vancouver, British Columbia V6E 3J7 (CA)
(74) Representative: Slingsby, Philip Roy
(86) International application number: CA9500501
(87) International publication number: WO9708099

(56) References cited:
- EP-A- 0 202 820
- EP-A- 0 470 518
- DE-A- 1 916 540
- US-A- 4 400 270

## Description

### FIELD OF THE INVENTION

This invention relates to a system for treatment of waste water with ultra-violet radiation to reduce microbiological contamination.

### BACKGROUND OF THE INVENTION

Modern potable water supply systems and waste water handling systems have long relied on various methods to remove microbiological contamination. In treatment of water, it is generally contamination by fecal coliforms, particularly *E*. *coli* bacteria, that is monitored and treated as the presence of these organisms indicates fecal contamination that can lead to rapid spread of disease through the water supply or receiving water body. It is still not uncommon to discharge waste water streams without any treatment at all, raising the question of increased ecological damage and fouling of water bodies and potable water supplies. Generally, in the past, the preferred method of eliminating or reducing micro-organism contamination in potable water supplies and in waste water handling systems where practised has been through the use of chlorination. Chlorination suffers from disadvantages in that it is expensive to administer, the dosage of chlorine must be carefully controlled to ensure proper and effective treatment of the water, and it is not particularly effective with water that contains a large amount of suspended particulate solids. In treating waste water streams, there is the potential of introducing harmful and carcinogenic organo-chlorides.

Other methods have been developed to treat microbiological contamination of water that do not involve the addition of a treatment agent. The most popular amongst these techniques is exposing water to ultra-violet radiation to kill any foreign organisms. A number of systems have been developed to deliver ultra-violet radiation to a liquid (cf e.g. EP-A-0 202 820).

When designing a system to deliver ultra-violet radiation to water, there are a number of factors to take into account. First, ultra-violet radiation, particularly when of low intensity, does not penetrate very deeply into water and therefore, it is necessary to expose a relatively thin film of water to the radiation to ensure that a lethal dose of radiation is delivered. Second, the effectiveness of ultra-violet radiation is reduced when treating murky water such as sewage effluent. Suspended, particulate solids that increase the turbidity of the water cannot be penetrated by the ultra-violet radiation.

The result of the foregoing design considerations is that previous ultra-violet water treatment systems have often been small scale systems that have a limited throughput. Examples of prior small scale systems for treating aquarium water or water as it emerges directly from the household tap are exemplified in United States Patent 2,338,387 to Whitman, United States Patent 3,731,090 to Veloz, and United States Patent 4,184,076 to Kosnoff.

United States Patent 4,336,223 to Hillman, United States Patent 3,711,709 to Rudolf and United States Patent 3,837,800 to Wood disclose larger scale systems for treating water with ultra-violet radiation. Wood is a good example of the design considerations that limit current ultra-violet sterilization systems. Wood discloses a system that is limited to thin sheet flow of water to be treated past an ultra-violet source to ensure adequate penetration of the ultra-violet radiation. To increase the dosage of radiation to a level that will kill microorganisms, Wood has to use a long residence time. The use of laminar sheet flow of water with a long residence time leads to a very limited capacity for Wood's system.

### SUMMARY OF THE INVENTION

The present invention provides an ultra-violet treatment system for water that addresses the low throughput problems of the prior art caused by the limited penetration of ultra-violet radiation through murky water.

The system of the present invention relies on a system of channels about a radiation source that create turbulent plug flow past the radiation source to greatly increase the throughput of the system. To do this, turbulent flow is established to create a cyclical flow of water past the radiation source so that water is continually transported from directly adjacent the radiation source to a position distant from the radiation source and back again to permit equal exposure of all water to a lethal radiation dose.

Accordingly, the present invention provides an apparatus for exposing a liquid to bactericidal radiation comprising:
an elongate source of ultra-violet radiation extending about a longitudinal axis;
non-opaque housing means enclosing the elongate source of ultra-violet radiation to prevent direct exposure of the source to the liquid and permit exposure of the liquid to the radiation;
channel means positioned to form uniform channels conforming about the housing means to confine and direct flow past the elongate source in a direction that is generally at right angles to the longitudinal axis of the elongate source of ultra-violet radiation; and
projections extending substantially parallel to the longitudinal axis of the elongate source of ultra-violet radiation for inducing turbulent flow in the channels such that when the apparatus is inserted into a flow of liquid to be treated the projections act to establish a cyclical flow in the channels between the housing means and the channel means at substantially right angles to the longitudinal axis of the elongate source to ensure balanced exposure of the liquid to a lethal dose of the ultra-violet radiation of the source.

In a further aspect, the present invention provides a method for exposing a liquid to bactericidal radiation comprising the steps of:
providing an elongate source of ultra-violet radiation having a longitudinal axis;
moving liquid to be treated past the source of the ultra-violet radiation in a direction at generally right angles to the longitudinal axis of the elongate source in paths conforming about the source; and
inducing a turbulent cyclical flow of the liquid at substantially right angles to the longitudinal axis in the paths about the radiation source to ensure balanced exposure of the liquid to the ultra-violet radiation of the source.

The apparatus and method of the present invention provide a system for sterilizing water using ultra-violet radiation that offers improved efficiency, capacity and performance over the prior art.

### BRIEF DESCRIPTION OF THE DRAWINGS

Aspects of the present invention are illustrated, merely by way of example, in the accompanying drawings, in which:
Figure 1 is a perspective view of the system of the present invention in place in a waste water flow channel;
Figure 2 is a plan view of the ultra-violet radiation source and associated flow passage of the present invention slowing the flow path of liquid through the passage;
Figure 3 is a partial plan view showing an alternative flow path through the passages;
Figure 4 is a plan view of a plurality of the units shown in Figure 2 arranged in series;
Figure 5 is a plan view of a plurality of the units shown in Figure 2 arranged in parallel;
Figure 6 is a perspective view of the apparatus of Figure 1; and

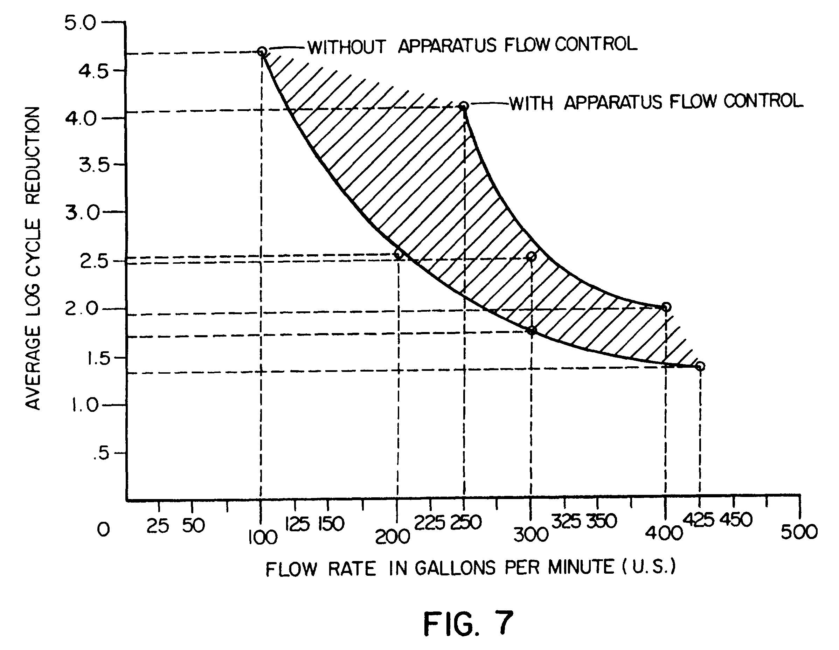

Figure 7 is a graph showing the effectiveness of the apparatus of the present invention in reducing bacterial infection as compared to prior art methods over a range of flow rates.

### DESCRIPTION OF THE PREFERRED EMBODIMENTS

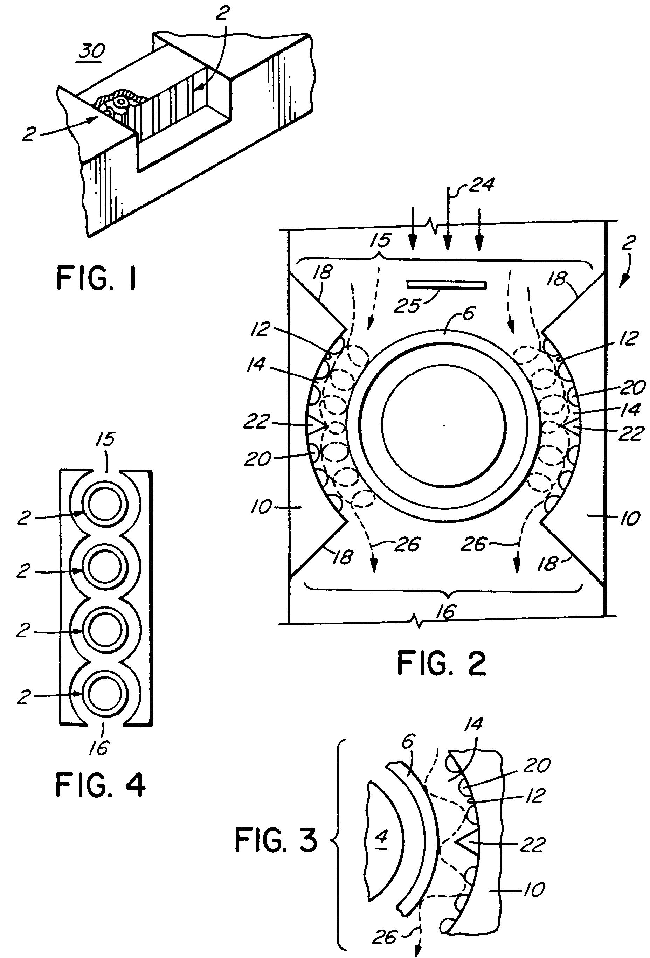

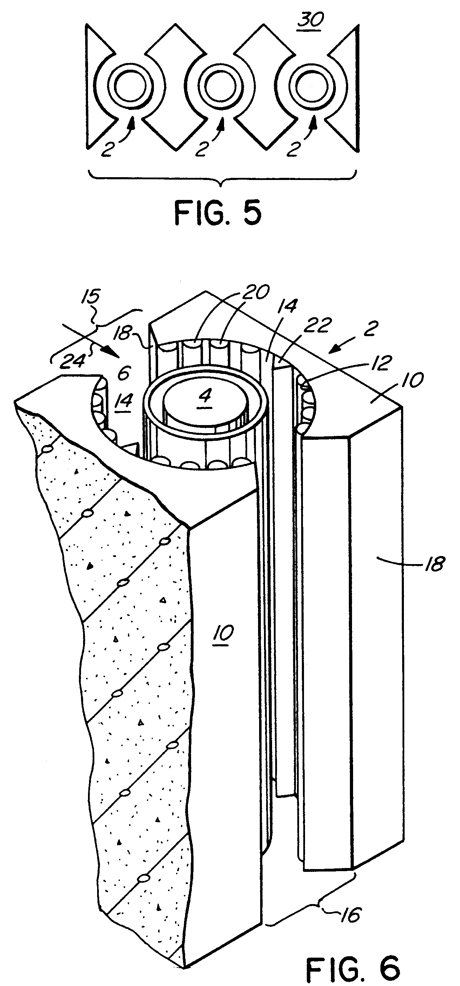

Referring to Figure 2 and 5, there is shown apparatus 2 for exposing a liquid water to ultra-violet radiation according to a preferred embodiment of the present invention. The apparatus comprises a source of ultra-violet radiation in the form of an elongated, high intensity fluorescent tube 4. When tube 4 is plugged into a electric source it generates the desired ultra-violet radiation. Tube 4 is a high intensity source of ultra-violet radiation generating wavelengths in the range of 254 rim. Tube 4 is enclosed within a non-opaque housing 6 that prevents direct exposure of the radiation source to any liquid passing through the apparatus while permitting exposure of the liquid to the radiation. Housing 6 is a transparent quartz sheath that transmits virtually all the ultra-violet radiation generated by tube 4.

Housing 6 is partially surrounded on either side by channel means positioned to form uniform width channels of semi-circular configuration that conform about housing 6. The channel means comprises a pair of spaced, elongate wall members positioned adjacent and parallel housing 6 on opposite sides thereof. Each elongate wall member 10 is formed with a concave inner wall 12 to define a uniform, set-annular channel 14 between the housing 6 and the wall 12 for liquid flow on either side of the housing 6. The entrance 15 and the exit 16 to channels 14 are preferably formed with inwardly angled walls 18 that co-operate to guide liquid flow through channels 14.

Within channels 14 are provided means for inducing turbulent flow in the channels. The flow through the channels is plug flow in which all fluid elements move at essentially the same rate. The means for inducing turbulent flow comprises a plurality of projections 20 and 22 that extend into the channels from the concave inner walls 12. Set-circular projections 20 and triangular projection 22 are designed to induce turbulent, cyclical flow in the liquid flowing through channels 14. Semi-circular projections 20 act to break up the boundary layer of liquid adjacent inner walls 12 and move the liquid toward the radiation source. Triangular projections are positioned to direct flow at the radiation source and project 1/3 of the distance from wall to radiation source. As best shown in Figure 2, liquid to be treated enters the apparatus through entrance 15, as indicated by arrow 24, and splits to enter one of the channels 14. A barrier 25 can be inserted in entrance 15 to assist in splitting of the liquid flow and establishing turbulent flow. Once within channels 14, the liquid is caused to move in a cyclical pattern. For example, as indicated by dashed arrow 26, liquid flow will tend to move in a helical pattern, or as shown in Figure 3, flow can also be established in a sinuous pattern.. In practice, flow will be a combination of these general cycle patterns. This cyclic flow of the liquid from channel wall-to- radiation source-to-channel wall ensures that the liquid is exposed to the average of the maximum radiation intensity at the quartz sheath and the minimum radiation intensity at the channel wall. Therefore, all elements of the liquid are equally exposed and tend to receive a lethal radiation dose to kill micro-organisms. This allows for optimum use of the ultra-violet radiation and optimum treatment efficiency.

The cyclical flow arrangement of the present invention also permits the apparatus to handle a greater liquid throughput and a liquid having a greater turbidity or murkiness than in the past. Intensity of ultra-violet light falls off exponentially with distance from the source and is further compounded by an exponential fall off with reduced transmission through the liquid. With a typical ultra-violet transmission of 20% in sewage effluent, the ultra-violet intensity 0,635 cm (0.25 inches) from the source would be reduced to about 35% of the source intensity, at which point the bactericidal effect of the radiation is significantly reduced. Therefore, prior art systems have relied on very, thin laminar flow and long residence time past an ultra-violet radiation source to ensure that the liquid received a lethal dose of radiation.

In contrast, the system of the present invention with its turbulent, cyclical flow can treat a greater depth of liquid. In a prototype test, a channel width of 3,175 cm (1.25 inches) was used. In this arrangement, the ultra-violet intensity at the channel wall for a liquid such as sewage having an ultra-violet transmission of 20% is essentially negligible at 0.6% of the source intensity. The apparatus of the present invention, however, continuously cycles the fluid from the channel wall (at 0.6% intensity) to the quartz sheath (at nearly 100% intensity) thus giving the liquid an average intensity exposure of nearly 50%. The sterilizing effect of such an exposure is demonstrated by a bacterial kill of 3 to 5 log cycle reductions in prototype testing using the extreme case of an actual mix of raw sewage as the liquid to be treated. The results of the prototype testing are set out below.

As well as providing a greater average exposure intensity leading to significant bacterial kill, the wider channels of the present invention permit a greater liquid throughput.

The apparatus of Figures 2 and 5 represents a basic unit for incorporation into an array of units as illustrated in Figures 1, 3 and 5 to treat larger volumes of liquid. The unit 2 of Figure 2 can be arranged in an array of interconnected units that define a plurality of parallel channels to extend across a passage 30 for liquid flow as illustrate in Figure 4. Alternatively, the units 2 can be arranged as in Figure 3 in an array of interconnected units to give multiplied efficiency that define a plurality of channels organized in series in which the exit 16 of one unit is the entrance 15 of the next. The most useful arrangement is illustrated in Figure 1. This array is a combination of the arrays of Figure 3 and 4 in which the units 2 are positioned in multiple rows of parallel channels across a passage for liquid flow 30. The units are dimensioned to be the same height as the passage 30 to handle any level of liquid flow. Passage 30 can be an open waste water channel from a sewage treatment plant or the like, or a sealed channel.

The improved efficiency, capacity and performance of the apparatus and method of the present invention are apparent in the following summary of data collected during stringent prototype testing of the present invention. The testing was carried out using a single ultra-violet source and a channel width of 5,08 cm (2 inches) between the quartz sheath and the channel wall-a-width designed to stretch the limits of the invention. Various flow rates of sewage effluent having an average transmittance of ultra-violet radiation of 50% were run through the prototype. *E*. *coli* counts were made before and after treatment. For comparison purposes, the same tests were run on a ultra violet unit having a radiation source positioned between parallel, planar side walls positioned 5,08 cm (2 inches) on either side of the radiation source.

### Summary of Data collected using Unit according to the present invention

| Flow ℓ/min (US gal/min) | Mean No. *E*. *coli*/100ml | | |
|---|---|---|---|
| | Influent | Effluent | Mean Log Cycle Reduction |
| 946 (250) | 53,000 | 33 | 4.06 |
| 1135 (300) | 60,000 | 283 | 2.47 |
| 1514 (400) | 83,000 | 1200 | 1.91 |

### Summary of Data collected using Unit having planar side walls

| Flow ℓ/min (US gal/min) | Mean No. *E*. *coli*/100ml | | |
|---|---|---|---|
| | Influent | Effluent | Mean Log Cycle Reduction |
| 378,5 (100) | 46,700 | 0 | 4.67 |
| 757 (200) | 56,700 | 367 | 2.52 |
| 1135 (300) | 36,700 | 700 | 1.71 |
| 1514 (400) | 33,300 | 1567 | 1.33 |

Figure 6 is a plot of flow rate of liquid against the average log cycle reduction in fecal bacteria based on the results shown in the above tables. The results demonstrate the superior lethality of the apparatus and method of the present invention at higher flow rates. For example, at 946 ℓ/min (250 US gallons per minute), with the sewage effluent ultra-violet transmission at approximately 50%, the parallel sided system gave a 2 log cycle reduction of bacteria (reduction by a factor of 100) while the apparatus of the present invention gave a 4 log cycle reduction (reduction by a factor of 10,000).

Although the present invention has been described in some detail by way of example for purposes of clarity and understanding, it will be apparent that certain changes and modifications may be practised within the scope of the appended claims.

## Claims

1. Apparatus for exposing a liquid to bactericidal radiation comprising:
an elongate source of ultra-violet radiation (4) extending about a longitudinal axis;
non-opaque housing means (6) enclosing the elongate source of ultra-violet radiation to prevent direct exposure of the source to the liquid and permit exposure of the liquid to the radiation;
channel means (14) positioned to form uniform channels conforming about the housing means to confine and direct flow past the elongate source in a direction that is generally at right angles to the longitudinal axis of the elongate source of ultra-violet radiation; and
projections (20) extending substantially parallel to the longitudinal axis of the elongate source of ultra-violet radiation for inducing turbulent flow in the channels such that when the apparatus is inserted into a flow of liquid to be treated the projections act to establish a cyclical flow in the channels between the housing means and the channel means at substantially right angles to the longitudinal axis of the elongate source to ensure balanced exposure of the liquid to a lethal dose of the ultra-violet radiation of the source.

2. Apparatus as claimed in claim 1 in which the source of ultra-violet radiation is a lamp having an elongate, cylindrical configuration.

3. Apparatus as claimed in claim 2 in which the ultra-violet source is a medium or high intensity source producing light that includes wavelengths of 254nm.

4. Apparatus as claimed in claim 2 in which the non-opaque housing means comprises a transparent, quartz sheath.

5. Apparatus as claimed in claim 4 in which the channel means comprise a pair of spaced elongate members positioned adjacent and parallel the housing means on opposite sides thereof, each elongate member having a concave inner wall to define a uniform width, annular channel between the housing means and the wall for liquid flow on either side of the housing means.

6. Apparatus as claimed in claim 1 in which the width of the channels ranges from 0.019 m to 0,05 m (0.75 inches to 2 inches).

7. Apparatus as claimed in claim 1 in which the projections extend into the channels from the channels means.

8. Apparatus as claimed in claim 1 arranged in an array of interconnected units to define a plurality of parallel channels insertable into a passage for liquid flow to extend across the passage.

9. Apparatus as claimed in claim 1 arranged in an array of interconnected units to define a plurality of channels arranged in series to receive liquid flow.

10. Apparatus as claimed in claim 1 arranged in an array of interconnected units to define multiple rows of parallel channels insertable into a passage for liquid flow to extend across the passage.

11. Apparatus as claimed in claim 1 in which the projections establish a helical flow of liquid at substantially right angle to the longitudinal axis of the source.

12. Apparatus as claimed in claim 1 in which the projections establish a sinuous flow of liquid at substantially right angles to the longitudinal axis of the source.

13. Method for exposing a liquid to bactericidal radiation comprising the steps of:
providing an elongate source of ultra-violet radiation having a longitudinal axis;
moving liquid to be treated past the source of the ultra-violet radiation in a direction at generally right angles to the longitudinal axis of the elongate source in paths conforming about the source; and
inducing a turbulent cyclical flow of the liquid at substantially right angles to the longitudinal axis in the paths about the radiation source to ensure balanced exposure of the liquid to the ultra-violet radiation of the source.

14. The method of claim 13 in which movement of the liquid to be treated past the radiation source in paths conforming about the source is accomplished by forming uniform channels about the radiation source to guide and direct the flow of liquid.

15. The method of claim 13 including providing a non-opaque housing about the radiation source to protect the source from exposure to liquid.

16. The method of claim 13 in which the cyclical flow is established by providing turbulence inducing projections in the paths that extend substantially parallel to the longitudinal axis of the elongate source.

17. The method of claim 13 in which a plurality of radiation sources are arranged in parallel rows across a liquid flow to accommodate simultaneous treatment of a large volume of liquid.

## Patentansprüche

1. Vorrichtung, die dazu dient, eine Flüssigkeit bakterizider Strahlung auszusetzen, mit:
- einer länglichen Ultraviolettstrahlungsquelle (4), die sich entlang einer Längsachse erstreckt;
- einer undurchsichtigen Gehäuseeinrichtung (6), die die längliche Ultraviolettstrahlungsquelle umschließt, um zu verhindern, dass die Quelle der Flüssigkeit direkt ausgesetzt ist, und um zu ermöglichen, dass die Flüssigkeit der Strahlung ausgesetzt ist;
- einer Kanaleinrichtung (14), die so positioniert ist, dass sie um die Gehäuseeinrichtung herum passende gleichmäßige Kanäle aufweist, um eine Strömung entlang der länglichen Quelle in einer Richtung einzuschließen und zu leiten, die im Wesentlichen rechtwinklig zur Längsachse der länglichen Ultraviolettstrahlungsquelle verläuft; und
- Vorsprüngen (20), die sich im Wesentlichen parallel zur Längsachse der länglichen Ultraviolettstrahlungsquelle erstrecken, um in den Kanälen eine turbulente Strömung hervorzurufen, so dass dann, wenn die Vorrichtung in eine Strömung zu behandelnder Flüssigkeit eingetaucht wird, die Vorsprünge so wirken, dass sie in den Kanälen zwischen der Gehäuseeinrichtung und der Kanaleinrichtung eine Ringströmung im Wesentlichen rechtwinklig zur Längsachse der länglichen Quelle aufbauen, um dafür zu sorgen, dass die Flüssigkeit in ausgeglichener Weise einer tödlichen Dosis der Ultraviolettstrahlung der Quelle ausgesetzt wird.

2. Vorrichtung nach Anspruch 1, bei der die Ultraviolettstrahlungsquelle eine Lampe mit länglicher, zylindrischer Form ist.

3. Vorrichtung nach Anspruch 2, bei der die Ultraviolettquelle eine Quelle mittlerer oder hoher Intensität ist, die Licht erzeugt, das Wellenlängen von 254 nm enthält.

4. Vorrichtung nach Anspruch 2, bei der die undurchsichtige Gehäuseeinrichtung einen transparenten Quarzmantel aufweist.

5. Vorrichtung nach Anspruch 4, bei der die Kanaleinrichtung ein paar beabstandete längliche Elemente aufweist, die angrenzend an die und parallel zur Gehäuseeinrichtung auf entgegengesetzten Seiten derselben angeordnet sind, wobei jedes längliche Element eine konkave Innenwand aufweist, um einen Ringkanal gleichmäßiger Breite zwischen der Gehäuseeinrichtung und der Wand für eine Flüssigkeitsströmung auf jeder Seite der Gehäuseeinrichtung zu bilden.

6. Vorrichtung nach Anspruch 1, bei der die Breite der Kanäle im Bereich von 0,019 in bis 0,05 in (0,75 Zoll bis 2 Zoll) liegt.

7. Vorrichtung nach Anspruch 1, bei der sich die Vorsprünge in den Kanälen von der Kanaleinrichtung aus erstrecken.

8. Vorrichtung nach Anspruch 1, die mit einem Array von miteinander verbundenen Einheiten angeordnet ist, um mehrere parallele Kanäle zu bilden, die in einen Durchlass für eine Flüssigkeitsströmung eintauchbar sind, um sich durch den Durchlass hindurch zu erstrecken.

9. Vorrichtung nach Anspruch 1, die mit einem Array von miteinander verbundenen Einheiten angeordnet ist, um mehrere Kanäle zu bilden, die seriell angeordnet sind, um eine Flüssigkeitsströmung zu erhalten.

10. Vorrichtung nach Anspruch 1, die mit einem Array von miteinander verbundenen Einheiten angeordnet ist, um mehrere Reihen paralleler Kanäle zu bilden, die in einen Durchlass für eine Flüssigkeitsströmung eintauchbar sind, um sich durch den Durchlass hindurch zu erstrecken.

11. Vorrichtung nach Anspruch 1, bei der die Vorsprünge eine schraubenförmige Flüssigkeitsströmung im Wesentlichen rechtwinklig zur Längsachse der Quelle erzeugen.

12. Vorrichtung nach Anspruch 1, bei der die Vorsprünge eine sinusförmige Flüssigkeitsströmung im Wesentlichen rechtwinklig zur Längsachse der Quelle erzeugen.

13. Verfahren, das dazu dient, eine Flüssigkeit bakterizider Strahlung auszusetzen, mit den folgenden Schritten:
- Bereitstellen einer länglichen Ultraviolettstrahlungsquelle mit einer Längsachse;
- Bewegen von zu behandelnder Flüssigkeit an der Ultraviolettstrahlungsquelle in einer Richtung im Wesentlichen rechtwinklig zur Längsachse der länglichen Quelle in Pfaden, die um die Quelle herum derselben entsprechen, vorbei; und
- Hervorrufen einer turbulenten Ringströmung der Flüssigkeit im Wesentlichen rechtwinklig zur Längsachse in Pfaden um die Strahlungsquelle herum, um dafür zu sorgen, dass die Flüssigkeit in ausgeglichener Weise einer tödlichen Dosis der Ultraviolettstrahlung der Quelle ausgesetzt wird.

14. Verfahren nach Anspruch 13, bei dem die Bewegung der zu behandelnden Flüssigkeit an der Strahlungsquelle vorbei in der Quelle entsprechenden Pfaden dadurch erfolgt, dass um die Strahlungsquelle herum gleichmäßige Kanäle ausgebildet werden, um die Flüssigkeitsströmung zu führen und zu leiten.

15. Verfahren nach Anspruch 13, mit dem Anbringen eines undurchsichtigen Gehäuses um die Strahlungsquelle herum, um die Quelle davor zu schützen, Flüssigkeit ausgesetzt zu sein.

16. Verfahren nach Anspruch 13, bei dem die Ringströmung dadurch aufgebaut wird, dass Turbulenz hervorrufende Vorsprünge in den Pfaden angebracht werden, die sich im Wesentlichen parallel zur Längsachse der länglichen Quelle erstreckten.

17. Verfahren nach Anspruch 13, bei dem mehrere Strahlungsquellen in parallelen Reihen durch eine Flüssigkeitsströmung hindurch angeordnet werden, um für gleichzeitige Behandlung eines großen Flüssigkeitsvolumens zu sorgen.

## Revendications

1. Appareil pour exposer un liquide à un rayonnement bactéricide, comportant :
une source allongée de rayonnement ultraviolet (4) s'étendant suivant un axe longitudinal ;
un moyen à enveloppe non opaque (6) renfermant la source allongée de rayonnement ultraviolet pour empêcher une exposition directe de la source au liquide et permettre une exposition du liquide au rayonnement ;
un moyen (14) à canaux positionné de façon à former des canaux uniformes se conformant autour du moyen à boîtier pour confiner et diriger un flux passant par la source allongée dans une direction qui est globalement à angle droit avec l'axe longitudinal de la source allongée de rayonnement ultraviolet ; et
des saillies (20) s'étendant sensiblement parallèlement à l'axe longitudinal de la source allongée de rayonnement ultraviolet pour induire un écoulement turbulent dans les canaux, tel que, lorsque l'appareil est introduit dans un écoulement de liquide devant être traité, les saillies agissent de façon à établir un écoulement cyclique dans les canaux entre le moyen à enceinte et le moyen à canaux sensiblement à angle droit par rapport à l'axe longitudinal de la source allongée pour assurer une exposition équilibrée du liquide à une dose létale du rayonnement ultraviolet de la source.

2. Appareil selon la revendication 1, dans lequel la source de rayonnement ultraviolet est une lampe ayant une configuration cylindrique allongée.

3. Appareil selon la revendication 2, dans lequel la source ultraviolette est une source d'intensité moyenne ou élevée produisant une lumière qui comprend des longueurs d'onde de 254 nm.

4. Appareil selon la revendication 2, dans lequel le moyen à enceinte non opaque comprend une gaine transparente en quartz.

5. Appareil selon la revendication 4, dans lequel le moyen à canaux comporte deux éléments allongés et espacés, positionnés de façon à être adjacents et parallèles au moyen à enceinte sur des côtés opposés de celui-ci, chaque élément allongé ayant une paroi intérieure concave destinée à définir un canal annulaire de largeur uniforme entre le moyen à enceinte et la paroi pour l'écoulement d'un liquide sur chaque côté du moyen à enceinte.

6. Appareil selon la revendication 1, dans lequel la largeur des canaux est comprise entre 0,019 m et 0,05 m (0,75 inch et 2 inches).

7. Appareil selon la revendication 1, dans lequel les saillies s'étendent vers l'intérieur des canaux à partir du moyen à canaux.

8. Appareil selon la revendication 1, agencé en une rangée d'unités raccordées entre elles pour définir plusieurs canaux parallèles pouvant être insérés dans un passage pour un écoulement de liquide de façon à s'étendre en travers du passage.

9. Appareil selon la revendication 1, agencé en une rangée d'unités raccordées entre elles pour définir plusieurs canaux agencés en série pour recevoir un écoulement de liquide.

10. Appareil selon la revendication 1, agencé en une rangée d'unités raccordées entre elles pour définir des alignements multiples de canaux parallèles pouvant être insérés dans un passage pour un écoulement de liquide afin de s'étendre en travers du passage.

11. Appareil selon la revendication 1, dans lequel les saillies établissent un écoulement hélicoïdal du liquide sensiblement à angle droit par rapport à l'axe longitudinal de la source.

12. Appareil selon la revendication 1, dans lequel les saillies établissent un écoulement sinueux du liquide sensiblement à angle droit par rapport à l'axe longitudinal de la source.

13. Procédé pour exposer un liquide à un rayonnement bactéricide, comprenant les étapes dans lesquelles :
on utilise une source allongée d'un rayonnement ultraviolet ayant un axe longitudinal ;
on déplace le liquide devant être traité, devant la source du rayonnement ultraviolet dans une direction à peu près à angle droit par rapport à l'axe longitudinal de la source allongée suivant des trajets épousant le pourtour de la source ; et
on induit un écoulement cyclique turbulent du liquide sensiblement à angle droit par rapport à l'axe longitudinal dans les trajets autour de la source de rayonnement pour assurer une exposition équilibrée du liquide au rayonnement ultraviolet de la source.

14. Procédé selon la revendication 13, dans lequel un mouvement du liquide devant être traité passant devant la source de rayonnement dans des trajets se conformant autour de la source, est obtenu en formant des canaux uniformes autour de la source de rayonnement pour guider et diriger l'écoulement de liquide.

15. Procédé selon la revendication 13, comprenant l'utilisation d'une enceinte non opaque autour de la source de rayonnement pour protéger la source d'une exposition à un liquide.

16. Procédé selon la revendication 13, dans lequel l'écoulement cyclique est établi par la mise en place de saillies induisant une turbulence dans les trajets qui s'étendent sensiblement parallèlement à l'axe longitudinal de la source allongée.

17. Procédé selon la revendication 13, dans lequel plusieurs sources de rayonnement sont agencées en alignements parallèles en travers de l'écoulement de liquide pour permettre de traiter au même moment un grand volume de liquide.
